# EUROPÄISCHE PATENTSCHRIFT

(11) **EP 0 440 321 B1**
(45) Veröffentlichungstag und Bekanntmachung des Hinweises auf die Patenterteilung: **13.01.1999**
(21) Anmeldenummer: 91250021.2
(22) Anmeldetag: 29.01.1991
(51) Int. Cl.: C12N 15/12, A61K 38/00, C12N 15/70, C12N 1/21, C12N 5/10, C12P 21/02, C07K 14/00, C12P 21/08, A61K 39/395, G01N 33/53

(54) **Epididymis-spezifische Polypeptide und deren Verwendung**
Epididymis specific polypeptides and their use
Polypeptides spécifique d'épididymis et leur utilisation

(30) Priorität: 01.02.1990 DE 4002981; 30.11.1990 DE 4038189
(43) Veröffentlichungstag der Anmeldung: 07.08.1991
(60) Teilanmeldung: 98250131.4
(73) Patentinhaber: IHF INSTITUT FÜR HORMON- UND FORTPFLANZUNGSFORSCHUNG GmbH, 22529 Hamburg (DE)
(72) Erfinder: Ivell, Richard, W-2000 Hamburg 73 (DE); Kirchhoff, Christiane, W-2352 Wattenbek (DE)
(74) Vertreter: UEXKÜLL & STOLBERG

(56) Entgegenhaltungen:
- INT. J. ANDROL. Bd. 13, Nr. 2, April 1990, OXFORD, UK Seiten 155 - 167; C. KIRCHLOFF ET AL.: 'Cloning and analysis of mRNAs expressed specifically in the human epididymis'
- REPROD. NUTR. DEV. Bd. 28, Nr. 5, 1988, PARIS, FRANCE Seiten 1283 - 1299; S. FOURNIER-DELPECH ET AL.: 'A ram epididymal secretory protein shares common antigenic determinants with rat epididymal proteins and human seminal plasma proteins'

## Beschreibung

Die Erfindung betrifft neue, Epididymis-spezifische Polypeptide sowie ferner die für diese Polypeptide kodierenden DNA-Sequenzen und deren Verwendung zur Herstellung von Arzneimitteln zur Diagnose und Behandlung der männlichen Infertilität.

Derzeit sind ca. 15% aller Paare in der Bundesrepublik Deutschland ungewollt kinderlos und ihr Anteil nimmt ständig zu. Die Ursachen für die Unfruchtbarkeit liegen zu gleichen Teilen bei Mann und Frau. Während jedoch die Ursachen bei der Frau weitgehend diagnostizierbar sind, können beim Mann nur in ca. 30% der Fälle organische Ursachen festgestellt werden. Etwa ein Drittel der verbleibenden 70% lassen sich auf Oligospermie unbekannten Ursprungs zurückführen, während bei den restlichen Fällen nach dem bisherigen Wissensstand keine organisch-biochemisch manifestierbaren Ursachen vorliegen.

Bei der Reifung der Spermien nimmt der Epididymis (Nebenhoden) eine Schlüsselrolle ein. Er besteht beim Menschen aus einem einzigen 5 m langen Kanal, der sehr stark mäanderartig aufgewunden ist. Die im Hoden gebildeten, noch unreifen Spermien werden zum Epididymis transportiert und während einer 2 bis 3 Tage dauernden Passage einem Reifungsprozeß unterzogen, im Verlaufe dessen sie u.a. ihre Beweglichkeit erlangen.

Es ist demgemäß davon auszugehen, daß männliche Unfruchtbarkeit in zahlreichen Fällen auf Störungen der im Epididymis ablaufenden Reifungsprozesse beruht.

Obwohl die anatomische Feinstruktur des humanen Epididymis makroskopisch, mikroskopisch und elektronenmikroskopisch sehr gut beschrieben ist (vgl. A.F. Holstein: Morphologische Studien am Nebenhoden des Menschen, zwanglose Abhandlungen aus dem Gebiet der normalen und pathologischen Anatomie, **20**, 1-91 (1969)), sind dessen Proteinprodukte mit wenigen Ausnahmen (vgl. beispielsweise Tezon et al., Immunochemical localization of secretory antigens in the human epididymis and their association with spermatozon, Biology of Reproduktion, **32**, 591-597 (1985)) nur wenig erforscht. Fast alle Kenntnisse über dieses Organ stammen aus Arbeiten an Ratten, Mäusen, Hamstern, Ebern, Bullen oder gelegentlich Affen, wobei die Tier-spezifischen Unterschiede bekanntermaßen groß sind.

Die an verschiedenen Tierarten gewonnenen Ergebnisse zur Spermienreifung beim Durchtritt durch den Epididymis lassen sich wie folgt zusammenfassen (vgl. T.G. Cooper: The Epididymis, Sperm Maturation and Fertilisation, Springer Verlag, Berlin, (1986)):
(a) Begeißelung und Hyperaktivierung der Spermien.
(b) Kapazitation, d.h. die Bereitschaft, die Akrosomenreaktion durchzuführen, wobei Dekapazitationsfaktoren, bei denen es sich vermutlich um epididymale Polypeptide handelt, eine regelnde Rolle spielen.
(c) Veränderung der Oberflächenantigene der Spermien, um die Bindung zwischen Spermien und Eizelle zu begünstigen.
(d) Veränderung der Spermienmembran, um die Fusion mit dem Ei zu ermöglichen.

Das diese Prozesse auslösende Stoffwechselgeschehen im Epididymis ist selbst bei den untersuchten Tieren weitgehend ungeklärt. Es konnte jedoch anhand von Gelelektrophoresen gezeigt werden, daß im Epididymis einige Polypeptide zu der aus der Rete Testis stammenden Seminalflüssigkeit hinzukommen. Unter diesen sind bei der Ratte die Polypeptide (a) bis (e) von Cooper (a.a.O., Tabelle 20), in denen das sogenannte "acidic epididymal glycoprotein (AEG)" (Lea et al., (1978)) bzw. die Polypeptide B bis E von Brooks und Mitarbeiter (D.E. Brooks und J. Higgins, Characterization and androgen-dependence of proteins associated with lunimal fluid and spermatozoa in the rat epididymis, Journal of Reproduction and Fertility, **59**, 363-375, (1980)) enthalten sind. Die Ergebnisse dieser Modelle weisen darauf hin, daß die Polypeptide unter Androgeneinfluß stehen.

Die aus Tierversuchen gewonnen Ergebnisse lassen sich jedoch aufgrund der hohen Gewebe- und Spezies-Spezifität nicht auf den Menschen übertragen. Beispielsweise wurden die bei der Ratte wesentlichen epididymalen Polypeptide oder die sie kodierenden mRNA's in keinem anderen Gewebe und, abgesehen von der Maus, in keiner anderen Spezies gefunden (D.E. Brooks et al., Europ. J. Biochem., **161**, 13-18 (1986); J. Biolog. Chem., **261**, 4956-4961, (1986)).

Es ist daher Aufgabe der Erfindung, Mittel zu schaffen, mit deren Hilfe Störungen des Proteinstoffwechsels im Epididymis beim Menschen - und insbesondere männliche Infertilität - diagnostiziert und gegebenenfalls therapiert werden können.

Zur Lösung der Aufgabe wird das erfindungsgemäß Epididymis-spezifische Polypeptid humanen Ursprungs sowie Fragmente desselben und allelische Varianten mit gleicher Immunogenität vorgeschlagen.

Zur Verwendung bei der Diagnose ist ferner die DNA-Sequenz gemäß Sequenzprotokoll (Seq. ID-Nr. 2 ) sowie syngene Sequenzen oder Fragmente derselben, aber auch solche Nukleotidsequenzen, welche mit den zuvor genannten hybridisieren - insbesondere als Sonden - geeignet, wobei durch die Degeneration des genetischen Codes verursachte Abweichungen erfindungsgemäß eingeschlossen sind.

Die Isolierung und Identifikation Epididymis-spezifischer Polypeptide nach konventionellen Verfahren ist wegen der geringen Mengen des zur Verfügung stehenden Gewebes nicht möglich.

Im Rahmen der vorliegenden Erfindung ist es nunmehr erstmals gelungen, spezifische Sekretproteine des menschlichen Nebenhodens nachzuweisen, zu charakterisieren und ihre Herstellung mittels Verfahren auf der Basis rekombinanter DNA-Technologie oder chemischer Synthese zu ermöglichen.

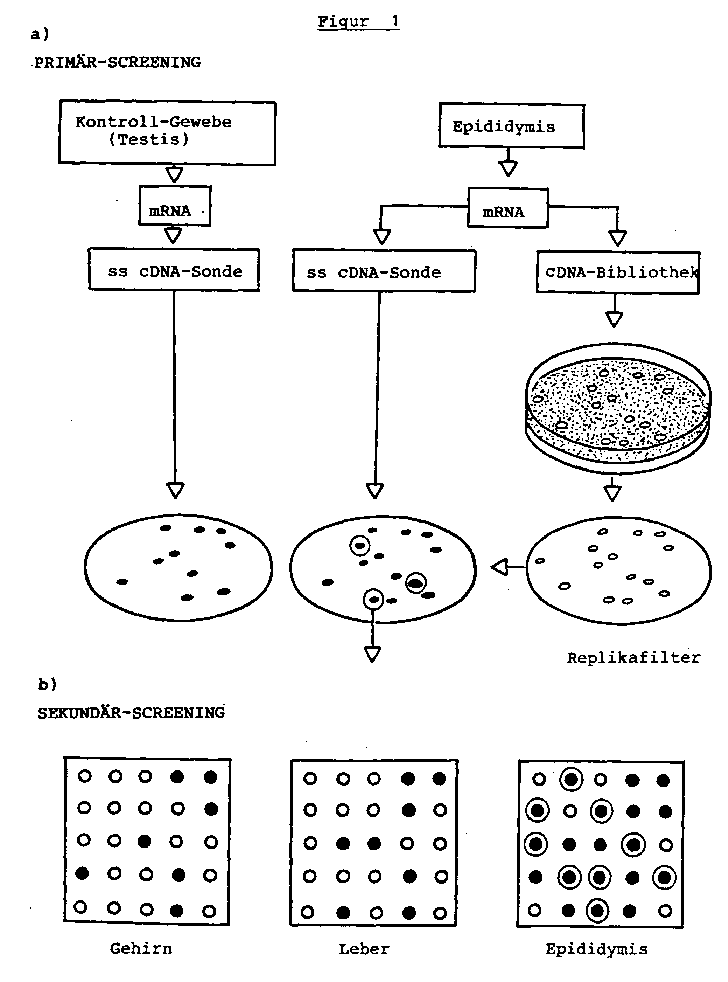

Zur Identifizierung der erfindungsgemäßen Polypeptide wurde zunächst eine cDNA-Bibliothek aus epididymaler mRNA in Lambda gt11 (Clontech, California) erstellt, sodann wurden die Epididymis-spezifischen Rekombinanten in einer komplexen Folge von differentiellen Hybridisierungsschritten selektiert (vgl. Figur 1) und anschließend in dem bakteriellen Plasmid pSB (Stratagene, California, USA) subkloniert und charakterisiert.

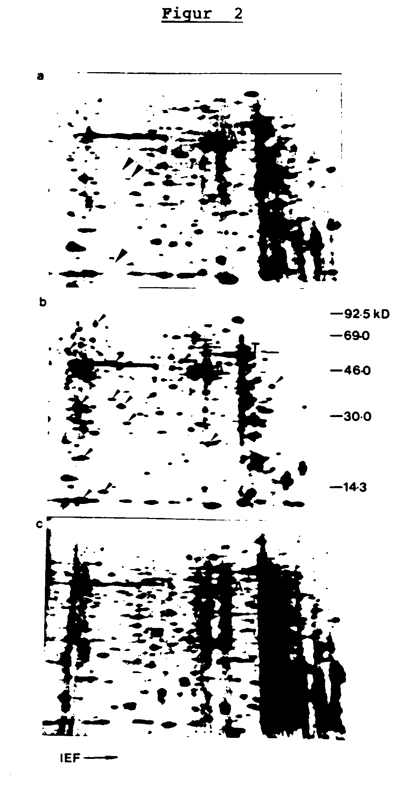

Das differentielle Screening zielte darauf ab, Klone von mRNA's zu isolieren, welche im Epididymis-Gewebe nicht aber im Gewebe anderer und funktionsverwandter Organe vorhanden sind oder dort nur in wesentlich reduzierter Kopienzahl auftreten. Bei dem erfindungsgemäß eingesetzten Verfahren diente im ersten Schritt menschliches Hodengewebe als Vergleichsmaterial. Da einige der als Ausgangsmaterial verwendeten Gewebeproben von orchiektomierten Männern stammten, die mit verschiedenen Medikamenten behandelt worden waren, mußte zunächst überprüft werden, ob und in welchem Umfang die Medikamentation Einfluß auf das jeweilige Polypeptidmuster ausübt. Es konnte jedoch an 7 Patienten unterschiedlichen Alters und unterschiedlicher Medikamentation gezeigt werden (vgl. Beispiel 2 und Figur 2a bis c), daß die Muster durch vorherige Verabreichung von Medikamenten nur unwesentlich beeinflußt werden und daß auch die individuellen Abweichungen gering sind.

Wie in Figur 1 schematisch dargestellt ist, erfolgte das Screening erfindungsgemäß in einer primären und einer sekundären Stufe, wobei in der primären Stufe durch Kreuzhybridisieren mit Testis-mRNA potentiell Epididymis-spezifische Klone isoliert wurden und diese in der sekundären Stufe durch Kreuzhybridisieren mit mRNA aus Gehirn und Leber weiter selektioniert wurden (vgl. Beispiel 4 und Figuren 3 und 4).

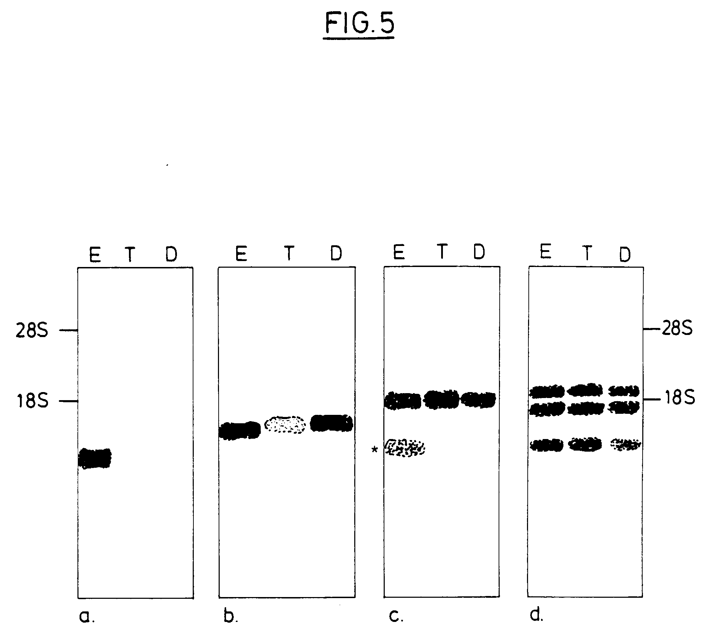

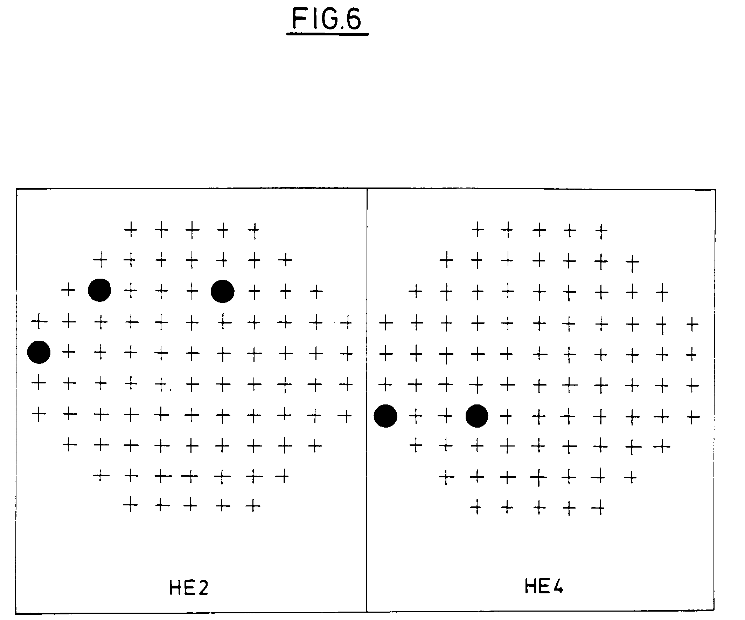

Durch nachfolgende Northern-Blot-Analyse gegen Gesamt-mRNA aus Hoden und menschlicher Decidua konnte die Zahl der in Betracht kommenden Klone weiter eingeengt werden und diese schließlich fünf unabhängigen cDNA-Familien zugeordnet werden, vgl. HE 1 bis HE 5, Beispiel 4 und Figuren 5 und 6, wobei der Klon HE 2 Gegenstand der vorliegenden Erfindung ist.

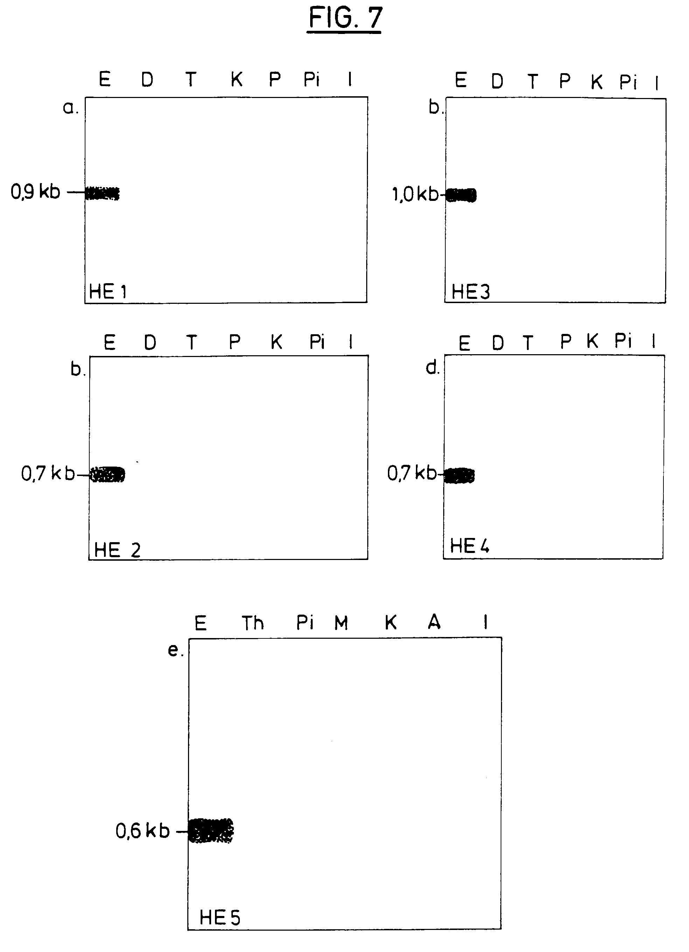

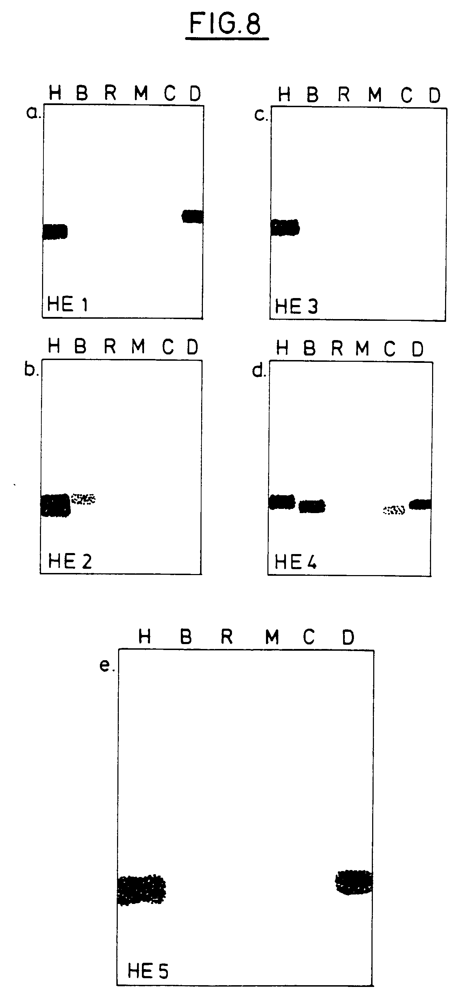

Die erfindungsgemäß gewonnenen Ergebnisse zeigen, daß die dem Klon HE 2 zugrunde liegende mRNA gar nicht oder nur sehr schwach in anderen menschlichen Geweben synthetisiert wird (vgl. Figur 7); es handelt sich demgemäß um ein Epididymis-spezifisches Molekül, das zugleich überwiegend Spezies-spezifisch ist (vgl. Fig. 8). Eine schwache Übereinstimmung zeigte sich lediglich beim Rind.

Die Figuren 5 bis 8 sind schematische Zeichnungen der jeweils zugrundeliegenden Autoradiogramme.

Die DNA-Sequenz des erfindungsgemäßen cDNA-Klones HE 2 (Seq. ID-Nr. 2) wurde wie in Beispiel 7 angegeben analysiert. Die Ergebnisse sind im Sequenzprotokoll wiedergegeben.

Die gefundene Sequenz (Seq. ID-Nr. 2) wurde auf Homologie zu den in verschiedenen internationalen Datenbanken (NIH-Genbank database, EMBL, PIR) gespeicherten Gensequenzen untersucht. Dabei zeigte es sich, daß es sich um ein bisher unbekanntes Gen handelt.

Das Vorhandensein einer tentativen Signalpeptidsequenz in HE 2, (Seq. ID-Nr. 2) zeigt, daß es sich um ein Gen handelt, welches für ein Sekretpolypeptid kodiert.

Der Klon HE 2 kann nach herkömmlichen Verfahren über geeignete Vektoren in pro- oder eukaryontische Wirtszellen transferiert und dort als Protein exprimiert werden.

Erfindungsgemäß sind alle jene DNA-Sequenzen geeignet und eingeschlossen, die nach Transformation geeigneter pro- oder eukaryontischer Wirtszellen die Gewinnung von Nukleinsäuren zur Verwendung als Diagnostika und/oder die Expression von Polypeptiden gewährleisten, welche zumindest einen Teil der jeweiligen Primärstruktur und eine oder mehrere der biologischen oder immunogenen Eigenschaften der erfindungsgemäßen Polypeptide aufweisen. Diese Sequenzen in einzel- oder doppelsträngiger Form schließen insbesondere ein:
a) Die im Sequenzprotokoll unter den Seq. ID-Nr. 2 angegebene Nukleotidsequenz oder syngene Sequenzen oder Fragmente derselben sowie deren komplementäre Sequenzen;
b) DNA-Sequenzen, die mit den Protein-kodierenden Bereichen der unter a) angegebenen Nukleotidsequenzen
   hybridisieren, beispielsweise unter den in den Beispielen 3 oder 4 angegebenen Hybridisierungsbedingungen;
c) DNA-Sequenzen, die mit Ausnahme der durch die Degeneration des genetischen Codes verursachten Abweichungen, mit den unter a) und b) genannten Sequenzen hybridisieren.

Der Begriff "syngene Sequenz" umfaßt alle Sequenzen, die sich von dem gleichen oder homologen Gen ableiten und für die Polypeptide im Sinne der Erfindung kodieren bzw. zur Herstellung von Sonden verwendbar sind.

Die Erfindung umfaßt ferner natürlich vorkommende allelische Variationen des erfindungsgemäßen Polypeptides oder Fragmente derartiger Allelvariationen, wobei sich die verschiedenen allelischen Formen sowohl jeweils voneinander, als auch von der im Sequenzprotokoll unter den Seq. ID-Nr. 2 angegebenen Aminosäuresequenz hinsichtlich der jeweiligen Sequenzlänge als auch in Bezug auf Deletionen, Substitutionen, Insertionen oder Additionen von Aminosäuren unterscheiden können.

Als geeignete Vektoren für prokaryontische Wirtszellen kommen beispielsweise Plasmide der pET-Serie (Rosenburg et al., "Vectors for selective expression of cloned DNAs by T7 RNA Polymerase", Gene, 56, S. 125-135 (1987)), der pGEX-Serie (Pharmacia, Freiburg) und der pRIT-Serie (Pharmacia, Freiburg) in Betracht. Beispiele für prokaryontische Wirtszellen sind die üblichen Laborstämme von Escherichia coli K12 sowie die Stämme BL21 (DE3) und LE392.

Beispiele geeigneter Vektorsysteme für eukaryontische Wirtszellen sind im Falle von Säuger-Zellen SV40 Viren, Polyoma-Viren, Adenoviren, verschiedene Retroviren, Papillomaviren (P.W.J. Rigby, "Expression of cloned genes in eucaryotic cells using vector systems derived from viral replicons", Genetic Engineering, Bd. 3, S. 84-141 (1982)), sowie Vaccinia-Viren (Mackett et al., "The construction and characterization of Vaccinia Virus recombinants expressing foreign genes", DNA Cloning, Bd. II, (1985), IRL Press, Oxford), und Derivate derselben sowie solche Plasmide, die Teile viraler Gene aufweisen (z.B. pSV2) und als "Shuttle-Vektoren" eingesetzt werden können (P.W.J. Rigby, a.a.O.). Als Vektoren für Insekten-Zellen kommen beispielsweise pJVETL-Bakuloviren (Invitrogen, San Diego, Calif., USA) in Betracht, während für Hefe-Zellen z.B. pJP31, YEp- und YIp-Plasmide (Carter et al., "Expression and secretion of foreign genes in Yeast", DNA Cloning, Bd. III, (1987), IRL Press, Oxford) verwendet werden können.

Geeignete Säuger-Zellen sind z.B. COS-Zellen, CHO-Zellen, AtT20-Zellen und NIH3T3-Zellen (Rigby, a.a.O., Mackett et al., a.a.O.), während geeignete Insekten-Zellen z.B. Sf9 (Invitrogen, San Diego, Calif., USA) sind. Geeignete Hefe-Zellen sind z.B. X4003-5B (Carter, a.a.O.).

Ferner kann das dem Klon HE 2 entsprechende Polypeptid nach bekannten Verfahren chemisch synthetisiert werden (J.M. Stewart, "Synthesis and use of neuropeptides", Neuroendocrine Peptide Methodology, ed. P.M. Conn, Academic Press, New York, S. 815-844 (1989))). Das gleiche gilt für Polypeptide oder Peptidepitope mit gleicher Immunogenität, die von Fragmenten oder syngenen Sequenzen der erfindungsgemäßen DNASequenz (Seq. ID-Nr. 2) kodiert sind.

Die erfindungsgemäße Nukleotidsequenz (Seq. ID-Nr. 2) einschließlich ihrer komplementären Sequenzen, ihre Expressionsprodukte sowie auf deren Basis hergestellte Antikörper bieten erstmals die Möglichkeit, Störungen im Proteinstoffwechsel des Epididymis zu diagnostizieren und gegebenenfalls zu therapieren.

So kann beispielsweise die cDNA-Sequenz oder deren komplementäre Sequenzen oder Fragmente derselben mit Markern versehen und als Sonde zur in situ-Hybridisierung bei der Gewebediagnostik von Biopsieproben oder Dünnschnitten verwendet werden, um den physiologischen Zustand des Gewebes hinsichtlich vorhandener Proteine zu bestimmen.

Ferner sind in diesem Zusammenhang markierte Antikörper einsetzbar, die von den erfindungsgemäßen Polypeptiden oder Fragmenten derselben induziert und in der Lage sind, eine Immunreaktion mit den entsprechenden Gewebeproteinen einzugehen.

Die auf der Basis der Sequenzen in prokaryontischen oder eukaryontischen Wirtszellen exprimierten oder synthetisch hergestellten Polypeptide sowie deren allelische Varianten mit gleicher Immunogenität oder Fragmente derselben können ferner in markierter oder unmarkierter Form als Antigene zur Identifizierung von Autoantikörpern in den Seren infertiler Männer dienen. Diese Möglichkeit ist von besonderer Bedeutung, da vermutet wird, daß die Infertilität in einem großen Teil der Fälle auf das Vorhandensein von Autoantikörpern gegen wesentliche Komponenten des Fortpflanzungssystems zurückzuführen ist. Die bisher zur Verfügung stehenden Testmethoden messen jedoch nur Antikörper, die gegen einige Sperm-Oberflächen-Antigene gerichtet sind, wobei ein ausreichend hoher Titer der Antikörper vorhanden sein muß, um die Sperm-Agglutination erfolgen zu lassen. Es wird jedoch angenommen, daß Antikörper in weitaus niedrigeren Titern vorhanden sein und Infertilität verursachen können. Diese könnten mit den erfindungsgemäß hergestellten reinen Polypeptiden als Antigene erfaßt werden.

Ferner können die erfindungsgemäßen Polypeptide oder Fragmente derselben mit gleicher biologischer Wirksamkeit für die in vitro Behandlung von Spermien infertiler Männer zum Einsatz kommen.

Polyklonale und monoklonale Antikörper zur Verwendung in immunologischen Nachweisverfahren können mit Hilfe der erfindungsgemäßen hochreinen Polypeptide auf bekannte Weise hergestellt werden. Derartige Antikörper lassen sich auf der Basis der vollständigen Polypeptide sowie auf der Basis von Fragmenten und allelischen Varianten derselben herstellen, soweit diese die gleiche Immunogenität besitzen (vgl. Beispiele 8, 9).

Ausgehend von der cDNA-Sequenz bieten sich hinsichtlich der Gewinnung von Antigenen zur Herstellung von Antikörpern zwei unterschiedliche Verfahren an.

Zum einen läßt sich mit Hilfe eines Computers eine potentiell immunogene Region der jeweils interessierenden, von der entsprechenden cDNA-Sequenz abgeleiteten Proteinsequenz auswählen, welche einerseits relativ hydrophil ist und damit an der Außenseite des Proteinmoleküls liegt, andererseits aber nicht durch Bildung von Cystein-Doppelbrücken oder möglichen Glykosilierungsstellen in ihrer sterischen Konformation gestört wird. Dieser Peptidbereich wird anschließend gegebenenfalls zusammen mit flankierenden Aminosäuren synthetisiert, nachfolgend an Trägersubstanzen gekoppelt und als Immunogen für die Induktion von Antikörpern eingesetzt (vgl. Beispiel 9).

Alternativ läßt sich beispielsweise das interessierende cDNA-Insert insgesamt oder aber ein großer Bereich davon in einen geeigneten Expressions-Plasmidvektor umklonieren. Nach anschließender Transformation in geeignete Bakterien erlauben diese Vektoren eine induzierbare Expression des von dem cDNA-Molekül kodierten Proteins. Das auf diese Weise hergestellte bakteriogene Protein oder Proteinfragment kann nach Auf reinigung aus dem Bakterienextrakt direkt als Antigen für eine Immunisierung zur Induktion von Antikörpern verwendet werden (vgl. Beispiel 8).

Die Antikörper können mit einem Marker wie z.B. einem fluoreszierenden Molekül (Fluorophor) versehen und beispielsweise bei Spermproben eingesetzt werden, um bei Infertilität das Vorhandensein von Nebenhoden-spezifischen Proteinen auf der Spermoberfläche mit Hilfe der Immunfluoreszenz zu bestimmen.

Weiterhin bietet sich die Möglichkeit, die Antikörper in einem Immunassay mit Seminalplasmaproben einzusetzen, um die Funktion des Nebenhodens zu überprüfen.

Sie können ferner zur Isolierung der entsprechenden Polypeptide aus Körperflüssigkeiten beispielsweise mittels Säulenchromatographie dienen. Die Erfindung umfaßt demgemäß auch derartige aus natürlichen Quellen isolierte Polypeptide.

Schließlich können die erfindungsgemäßen Proteine und gegen diese gerichtete Antikörper zur Immunsterilisierung verschiedener Säuger eingesetzt werden.

Da das HE-2 Protein ausschließlich in männlichen Organen synthetisiert wird, ist es insbesondere zur Anwendung im weiblichen Organismus geeignet. Die dort gegen die Fremdproteine erzeugten Antikörper werden nur an Spermien haftende Nebenhodenproteine im weiblichen Genitaltrakt erkennen und zu deren Agglutination bzw. Inaktivierung, nicht aber zu unerwünschten Nebenwirkungen durch Reaktionen mit Proteinen des weiblichen Organismus führen.

Eine Verhütungsimmunisierung im männlichen Organismus durch Verabreichung von HE-2 Protein zur Auslösung einer Auto immunantwort, die sich im Bereich des Nebenhodens in Form einer Inaktivierung der ihn durchwandernden Spermien auswirkt, stellt eine weitere Anwendungsform dar.

Somit können die beschriebenen DNA-Sonden, erfindungsgemäßen Proteine sowie gegen diese gerichtete Antikörper bei der Erkundung der Physiologie und Pathologie nicht nur des humanen Nebenhodens, sondern auch der Spermien und deren Interaktion mit Oozyten, in allen Tierarten mit kreuzreagierenden Aktivitäten (z.B. Hund, Rind und Katze) eingesetzt werden.

Die Erfindung wird nachfolgend anhand von Beispielen im einzelnen erläutert.

### Beispiel 1

### RNA-Präparation

Gesamt-RNA wurde nach dem Verfahren von J.M. Chirgwin et al., Isolation of biologically active ribonucleic acid from sources enriched in ribonuclease, Biochemistry, **18**, 5294, (1979), aus den gefrorenen Geweben menschlicher Hoden (Testis) und Nebenhoden (Epididymis), die Männern mit Prostatakarzinom im Alter von 58 und 74 Jahren durch Orchiektomie entnommen worden waren, unter Einsatz von Guanidin-Isothiocyanat extrahiert und anschließend durch Cäsiumchlorid-Dichtegradienten-Zentrifugation gereinigt. Durch Verwendung einer Oligo(dT)-Cellulosesäule konnte Poly(A)⁺RNA affinitätschromatographisch angereichert werden, wie von H. Aviv & P. Leder, Purification of biologically active globin messenger RNA by chromatography on oligothymidylic acid-cellulose, Proc. Natl. Acad. Sci., USA, **69**, 1408-1412, (1972), beschrieben. Die RNA-Proben wurden mit Ethanol gefällt und nach Resuspendieren in sterilem Wasser in einer Konzentration von 1 µg/µl bei -80°C aufbewahrt.

### Beispiel 2

### In vitro-Translation und Charakterisierung der Produkte durch zweidimensionale Gelelektrophorese

0,5 bis 1 µg der Poly(A)⁺RNA von Nebenhoden und Hoden aus Beispiel 1 wurden jeweils in einem zellfreien System eines Retikulozyten-Lysats aus Kaninchen (New England Nuclear (NEN), Dreieich, BRD) in vitro-translatiert. Die Synthese wurde in Gegenwart von (³⁵S)-Methionin spezifische Aktivität >1000 Ci/mmol) durchgeführt. Die resultierenden Proteinprodukte wurden anschließend in einem zweidimensionalen Gel elektrophoretisch aufgetrennt (vgl. P.H. O'Farrel, High resolution two-dimensional electrophoresis of proteins, J.Biol. Chem., **250,** 4007-4021 (1975)). Für die erste Dimension wurde jeweils eine Menge von 350 000 cpm der mit (³⁵S)-Methionin markierten Translationsansätze auf das Gel aufgetragen und bei einer angelegten Spannung von 10 000 V.h isoelektrisch fokussiert. Der pH-Gradient lag zwischen 4,0 und 7,5. Die Auftrennung der Polypeptide nach ihrem Molekulargewicht wurde in der zweiten Dimension innerhalb eines linearen Gradienten von 7,5 - 15% Acrylamid durchgeführt (vgl. D.M. Neville & H. Glossmann, Molecular weight determination of membrane protein and glycoprotein subunits by discontinous gel electrophoresis in dodecylsulfate, Meth. Enzym., **32**, 92-102, (1974)). Zur Abschätzung der Molekulargewichte wurde in der zweiten Dimension ein mit ¹⁴C markierter Protein-Marker (Amersham, GB) aufgetragen. Anschließend wurde das Gel mit Hilfe des autoradiographischen Films Kodak X-AR5 fluorographiert (vgl. W.M. Bonner & R.A. Laskey, A film detection method for Tritiumlabelled proteins and nucleic acids in polyacrylamide gels, Europ. J. Biochem., **46**, 83-88, (1974)). Die Expositionszeit betrug 8 Tage. Die Ergebnisse sind in Figur 2 wiedergegeben.

Diese zeigt das Muster der zellfrei synthetisierten Translationsprodukte, die sich von Nebenhoden- bzw. Hoden-spezifischer Poly(A)⁺RNA ableiten.

Insbesondere zeigt Figur
- 2a:: Nebenhodengewebe eines 74 Jahre alten, mit Cyproteronacetat behandelten Orchiektomie-Patienten,
- 2b:: Nebenhodengewebe eines 58 Jahre alten, nicht mit Medikamenten behandelten Orchiektomie-Patienten,
- 2c:: Hodengewebe desselben, nicht mit Medikamenten behandelten Patienten (siehe Figur 2b).

Nebenhoden-spezifische Translationsprodukte, deren korrespondierende Banden in Figur 2c (Translationsprodukte von mRNA aus Hoden) nicht auftauchen, sind durch kleine Pfeile gekennzeichnet (siehe Figur 2b).

Eine Veränderung der Expression im Nebenhodengewebe durch vorherige Behandlung mit Antiandrogenen zeigt ein Vergleich der Figuren 2a und 2b. Banden, die in Figur 2a gegenüber Figur 2b reduziert sind, wurden durch große Pfeile markiert. Die Molekulargewichte des Proteinmarkers sind in Kilodalton angegeben. Actin(A)- und Tubulin(T)-ähnliche Produkte sind jeweils gekennzeichnet.

### Beispiel 3

### Erstellung der cDNA-Gesamtbibliothek und Auswahl potentiell spezifischer Klone

Die Kultivierung, Handhabung und Erhaltung von Bakterien und Bakteriophagen sowie der Einsatz DNA-rekombinierender Techniken erfolgte nach den Angaben von T. Maniatis et al., Molecular Cloning, A Laboratory Manual, Cold Spring Harbour Laboratory, Cold Spring Harbour, N.Y., (1982).
a) Für die Erstellung der cDNA-Bibliothek wurde Poly(A)⁺RNA aus dem Nebenhoden-Gewebe eines nicht medikamentös behandelten Patienten eingesetzt (bezüglich Muster der in vitro-Translationsprodukte, vgl. Figur 2b). 20 µg von Poly(A)⁺RNA gemäß Beispiel 1 wurden unter Verwendung von Oligo(dT) als Primer revers transkribiert (vgl. U. Gubler & B.J. Hoffmann, A simple and very efficient method for generating cDNA libraries, Gene, **15**, 263-269, (1983)).
   Die doppelsträngigen cDNA-Konstrukte wurden auf beiden Seiten mit EcoRI-Linkern ligiert und anschließend in die EcoRI Klonierungsstelle des Bakteriophagen Lambda gt11 (Clontech California) eingeführt.
   Bakterien des E. coli-Stammes Y 1090 wurden bis zur logarhithmischen Phase angezogen, in Weich-Agarose überführt und zusammen mit der nicht amplifizierten Lambda-Bibliothek in einer Phagendichte von 500 bis 1000 pfu pro Petrischale (15 cm) ausplattiert und 8 Stunden lang bei 42°C inkubiert.
   Für das differentielle Screening wurden die Plaques einer jeden Schale nachfolgend auf zwei Nitrocellulose-Filter (Schleicher & Schüll, Darmstadt, BRD, BA85) Replika-plattiert, wobei die Inkubationszeit für das erste Filter 1 Minute, für das zweite Filter 2 Minuten betrug.
   In dieser Weise wurden Replika-Filter von insgesamt 20 Petrischalen hergestellt und anschließend mit jeweils zwei einzelsträngigen, radioaktiv markierten cDNA-Sonden (vgl. b) unten) (positiv/negativ) hybridisiert.
b) Die Sonden aus Poly(A)⁺RNA von Nebenhoden (liefert positive Sonde) und aus Poly(A)⁺RNA von Hoden (liefert negative Sonde) wurden unter Verwendung von Oligo(dT) als Primer wie folgt hergestellt:
   Beide RNA-Spezies entstammten den entsprechenden Geweben eines Patienten, der keiner medikamentösen Vorbehandlung ausgesetzt worden war. Jeweils 1 µg der Poly(A)⁺RNA gemäß Beispiel 1 wurde 5 Minuten lang bei 65°C in einem Volumen von 2 µl denaturiert. Nach schnellem Abkühlen der Ansätze auf Eis wurden jeweils folgende Bestandteile nacheinander zugegeben:
   - 2 µl Oligo(dT) (100 µg/ml)
   - 1 µl dNTP-Mix (dATP, dGTP, dTTP jeweils 2 mM)
   - 1 µl 40 mM Natriumpyrophosphat
   - 1 µl RNasin (Amersham; GB)
   - 2 µl 10x Reverse Transkriptase-Puffer (500 mM Tris.-Cl (pH 8,5), 500 mM KCl, 100 mM MgCl₂, 100 µg/ml BSA, 10 mM EDTA, 10 mM Dithiotreitol)
   - 20 Einheiten AMV reverse Transkriptase (Boehringer Mannheim, BRD)
   - 10 µl (α-³²P)-dCTP (NEN, 10 µ Ci/µl; S.A. > 3000 Ci/mmol).

Die Reaktionsansätze wurden 15 Minuten lang bei 42°C inkubiert. Nach Zugabe von 1 µl "Chasemix" (enthaltend alle vier dNTP's in einer Konzentration von jeweils 10 mM) wurde die Inkubation für weitere 20 Minuten fortgesetzt. Die Reaktion wurde durch Zugabe von jeweils 1 µl 0,5 M EDTA abgestoppt und die Produkte ohne weitere Reinigung mit Ethanol gefällt. Die mit einer spezifischen Aktivität von > 10⁸ cpm/µg markierten Sonden wurden mit den oben genannten Replika-Filtern parallel hybridisiert. Die Hybridisierung wurde in 5x Denhardt's-Lösung, 4x SET (200 mM Tris (pH 8,0), 20 mM EDTA, 0,6 M NaCl), 0,1% Natriumpyrophosphat und 25 mM Natriumphosphatpuffer (pH 7,0) 72 Stunden lang bei 65°C und einer Konzentration der Radioaktivität von 5 x 10⁶ cpm/ml durchgeführt. Die Filter wurden anschließend in 0,1% SDS, 2x SSC (300 mM Natriumchlorid, 30 mM Natrium₃-Citrat) bei einer Temperatur von 65°C gewaschen.

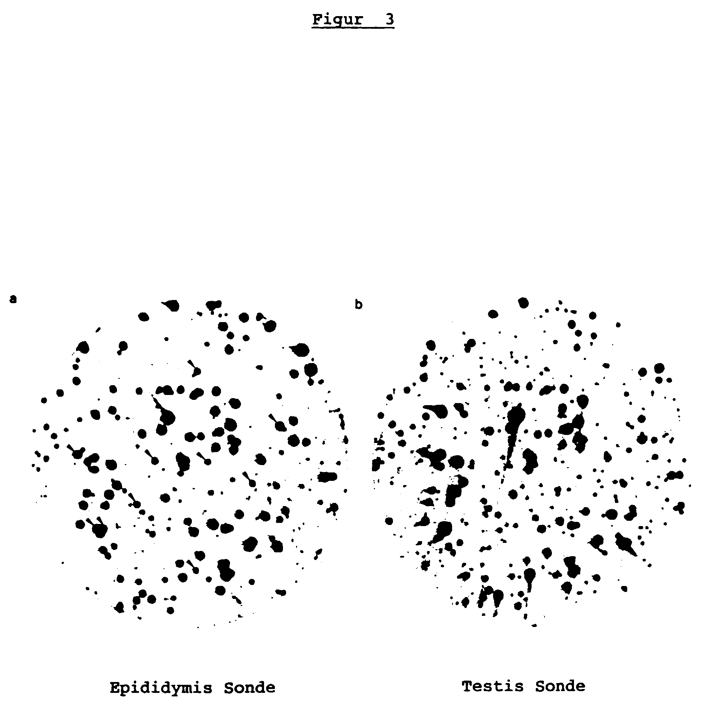

Figur 3 zeigt die Autoradiogramme zweier Replika-Filter von einer der Petrischalen, welche mit epididymalen (a) bzw. testikularen (b) cDNA-Sonden hybridisiert wurden. Die Entwicklung der Autoradiogramme erfolgte nach einer Expositionszeit von 16 Stunden unter Verwendung einer Verstärker-Folie. Die Filter repräsentieren etwa 500 unabhängige Klone der im System Lambda gt 11 erstellten cDNA-Gesamtbibliothek aus Nebenhodengewebe. Die positiven, Epididymis-spezifischen Hybridisierungssignale sind mit Pfeilen markiert (vgl. Figur 3a).

Mit Hilfe dieses Primär-Screening-Verfahrens (vgl. Figur 1a) konnten etwa 10 000 unabhängige cDNA-Klone analysiert werden. Dabei konnten 265 Rekombinanten identifiziert werden, deren Signale nach Hybridisierung mit der epididymalen cDNA-Sonde im Vergleich zu der aus Hodengewebe hergestellten sehr viel stärker waren. Bezogen auf den durch Screening analysierten Teil der Phagen-Bibiothek entspricht die Anzahl positiver cDNA-Klone einem Anteil von 2,5%.

Positive cDNA-Klone wurden isoliert und für ein Sekundär-Screening in 6 Gruppen mit bis zu 50 Klonen aufgeteilt.

Das oben beschriebene Verfahren wurde mit der Abweichung wiederholt, daß von jeder der 6 Petrischalen 3 Replika-Filter angefertigt wurden. Zur Herstellung der negativen cDNA-Sonden wurde hier Poly(A)⁺RNA aus menschlichem Gehirn bzw. aus menschlischer Leber (Clontech California, USA) verwendet.

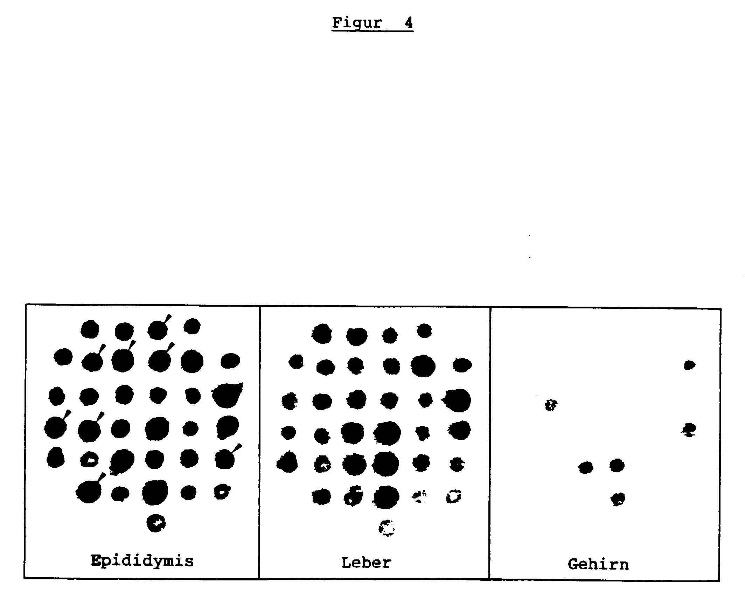

Die Anzahl positiver Rekombinanten wurde nach Analyse dieses sekundären Screening-Verfahrens (vgl. Figur 1b) auf 59 Klone reduziert (vgl. Figur 4).

Die Isolierung positiver Klone erfolgte ohne weitere Reinigung der Plaques. Die gereinigte Lambda-DNA wurde mit EcoRI geschnitten und die Inserts durch Biotrap-Elution (Schleicher & Schüll) isoliert. Die EcoRI-Inserts wurden in den bakteriellen Plasmid-Vektor pBS (Stratagene, California, USA) subkloniert und durch das CaCl₂-Transformationsverfahren in die Bakterienzellen des E.coli-Stammes XL1 Blue (Stratagene, California) eingeführt (vgl. T. Maniatis et al., Molecular cloning: A Laboratory Manual, Cold Spring Harbour Laboratory, Cold Spring Harbour, N.Y., (1982)).

### Beispiel 4

### Herstellung von Northern-Blots zur Analyse der gewebespezifischen Genexpression und Einteilung der Epididymis-spezifischen cDNA-Klone in Familien mit verwandten Sequenzen

20 µg Gesamt-RNA aus menschlicher Epididymis (E) von einem mit Cyproteronacetat behandelten Patienten sowie die gleiche Menge Gesamt-RNA aus menschlichem Hoden (T) und menschlicher Decidua (D) wurden in einem horizontalen 1,3%-igen Formaldehyd-Agarosegel 16 Stunden lang bei einer konstanten Spannung von 25 Volt elektrophoretisch aufgetrennt (vgl. Maniatis et al., Molecular Cloning: A Laboratory Manual, Cold Spring Harbour Laboratory, Cold Spring Harbour, N.Y., (1982) (Figur 3)). Zur Größenbestimmung der RNA's wurde das Gel zusätzlich mit einer RNA-Leiter (0,24-9,5 kB) beladen. Die RNA wurde anschließend durch Kapillar-Blotting in Gegenwart von 20 x SSC auf Nylonmembranen (Hybond N, Amersham) übertragen. Die Blots wurden nachfolgend mit radioaktiven, potentiell Epididymis-spezifischen cDNA-Sonden sukzessive hybridisiert. Die Sonden wurden durch EcoRI-Restriktion der im Plasmid pBS subklonierten Lambda gt11-DNA sowie anschließender Biotrap-Elution isoliert und wiesen nach radioaktiver Markierung eine spezifische Radioaktivität von > 10⁹ cpm/µg auf (vgl. A.P. Feinberg und B. Vogelstein, A technique for radiolabelling DNA restriction endonuclease fragments to high specific activity, Analytical Biochemistry, **132**, 6-13, (1983)). Die Hybridisierung erfolgte über Nacht bei einer Radioaktivitätskonzentration von 1-2 x 10⁶ cpm/ml. Nachfolgend wurden die Filter schrittweise unter steigender Stringenz gewaschen, beginnend mit 2 x SSC bei Raumtemperatur bis zu 0,1 x SSC bei einer Temperatur von 65°C. Zur Dehybridisierung wurden die Filter 15 Minuten bei 65°C in Gegenwart von 2 mM Tris (pH 7,5), 1 mM EDTA und 0,1% SDS inkubiert. Die getrockneten Filter wurden auf das Vorhandensein von Sondenmaterial autoradiographisch untersucht, bevor sie im Falle ausbleibender Signale für eine wiederholte Hybridisierung mit einer weiteren Sonde verwendet wurden.

In Figur 5 sind Beispiele für die Autoradiogramme der Northern-Blot-Analysen potentiell positiver cDNA-Klone dargestellt. Die Expositionszeit betrug 20 Stunden. Anhand der unterschiedlichen Hybridisierungsmuster wurden die Klone hinsichtlich ihrer Gewebespezifität qualitativ bewertet.
a) Positiver cDNA-Klon, angezeigt durch Epididymis-spezifische Hybridisierung.
b) Positives, aber nicht Epididymis-spezifisches Hybridisierungsmuster.
c) Positiver cDNA-Klon (*), der auch mit rRNA hybridisiert.
d) cDNA-Klon ohne Gewebe-spezifische Expression.

Mit Hilfe dieses Verfahrens konnte eine Anzahl von 36 epididymalen cDNA-Klonen identifiziert werden. Das spezifische Hybridisierungssignal eines jeden Klon-Inserts mit epididymaler RNA war um mindestens eine Größenordnung stärker als solche mit RNA aus Hoden oder Dezidua (vgl. Figur 5a).

Durch die Ergebnisse aus Kreuz-Hybridisierungen mit der ursprünglichen Phagenbibliothek konnten die 36 Epididymis-spezifischen cDNA-Klone fünf unabhängigen Familien (HE 1, HE 2, HE 3, HE 4 und HE 5) zugeordnet werden, die jeweils einen Satz zueinander verwandter Klone umfassen (vgl. Figur 6 für HE 2 und HE 4). Dabei konnte gezeigt werden, daß sich die fünf cDNA-Klon-Familien von fünf unterschiedlichen, relativ kurzen mRNA-Molekülen ableiten. Ihre ungefähren Längen sowie die anhand der Korrelation von Hybridisierungssignalen mit der Phagenbibliothek ausgezählten Häufigkeiten in bezug auf das Vorkommen einer jeden cDNA-Familiensequenz von HE 1 bis HE 5 sind in der Tabelle 1 dargestellt.

**Tabelle 1**

| Häufigkeit des Auftretens humaner Epididymis-spezifischer cDNA's und ungefähre Länge der mit ihnen korrespondierenden mRNA's | | |
|---|---|---|
| Genprodukt | Häufigkeit¹⁾ | Länge der mRNA (kB)²⁾ |
| HE 1 | 0,5% | 0,9 |
| HE 2 | 0,2% | 0,7 |
| HE 3 | 0,06% | 1,0 |
| HE 4 | 0,05% | 0,7 |
| HE 5 | 0,1% | 0,6 |

| | | |
|---|---|---|
| 1) Ungefähr 100.000 unabhängige Rekombinaten wurden für jede Sequenz einem Screening-Verfahren unterzogen. | | |
| 2) Zur Längenbestimmung der RNA-Moleküle wurde in der denaturierenden Elektrophorese eine RNA-Leiter mit einer Kettenlänge von 0,24 - 9,5 kB aufgetrennt. | | |

### Beispiel 5

### Kontrolle der Gewebespezifität der Klone HE 1 bis HE 5

Jeweils 8 µg Gesamt-RNA aus Epididymis (E), Decidua (D), Hoden (T), Prostata (P), Niere (K), Hypophyse (Pi) und der Lymphoblastom-Zellinie IM9 (1) wurden gemäß Beispiel 4 aufgetrennt und auf Nylonmembranen übertragen. Zur Kontrolle der Gewebespezifität von HE 5 wurden jeweils 8 µg Gesamt-RNA aus Epididymis (E), Schilddrüse (Th), Hypophyse (Pi), Muskel (M), Niere (K), Nebenniere (A) und der Lymphoblastom-Zellinie IM9 (I) analog aufgetrennt und ebenfalls auf eine Nylonmembran übertragen. Als Sonden wurden (³²P)-markierte cDNA-Inserts aus HE 1(a), HE 2(b), HE 3(c), HE 4(d) und HE 5(e) eingesetzt und mit Northern-Blots gemäß Beispiel 4 hybridisiert.

Die Ergebnisse für HE 1 bis HE 5 sind in Figur 7 dargestellt. Es zeigte sich, daß jeder der fünf cDNA-Klone nur mit der epididymalen RNA ein eindeutiges Hybridisierungssignal liefert.

### Beispiel 6

### Kreuz-Hybridisierungen zur Analyse der Spezies-spezifischen Genexpression

Jeweils 20 µg epididymale Gesamt-RNA aus Mensch (H), Rind (B), Ratte (R), Maus (M), Katze (C) und Hund (D) wurden gemäß Beispiel 4 aufgetrennt und auf Nylonmembranen übertragen. Als Sonden wurden (³²P)-markierte cDNA-Inserts aus HE 1(a), HE 2(b), HE 3(c), HE 4(d) und HE 5(e) eingesetzt und mit Northern-Blots gemäß Beispiel 4 hybridisiert, wobei jeweils der Vertreter mit der größten Kettenlänge eingesetzt wurde. In Figur 8 sind die nach einer Expositionszeit von 8 Stunden entwickelten Autoradiogramme der Northern-Blot-Analyse für die Klone HE 1 bis HE 5 schematisch dargestellt.

Bei dem cDNA-Insert aus dem Klon HE 3 konnte keine Kreuz-Hybridisierung mit tierischen RNA's festgestellt werden (vgl. Figur 8c), während das Insert aus dem Klon HE 2 lediglich eine sehr schwache Kreuz-Hybridisierung mit der Rinder-RNA zeigte (vgl. Figur 8b), wohingegen die Inserts aus den Klonen HE 1 und HE 5 starke Signale mit der epididymalen Hunde-RNA aufwiesen (vgl. Figur 8a, e). Da sich die Signale auch nach Waschen unter stringenten Bedingungen nicht reduzierten, liegen hier hohe Sequenzhomologien vor. Bei dem Insert des Klons HE 4 wurde ein starkes Signal mit epididymaler Rinder- und Hunde-RNA und ein sehr schwaches Signal mit der Katzen-RNA beobachtet (vgl. Figur 8d). Keine der menschlichen Sequenzen hybridisierte mit epididymaler RNA aus der Ratte und der Maus.

### Beispiel 7

### Bestimmung der Basensequenzen der Epididymis-spezifischen cDNA-Klone HE 1 bis HE 5

Die Basensequenz des jeweils längsten cDNA-Klons aus den Familien HE 1 bis HE 5 gemäß den Beispielen 4, 5 und 6 wurde mit Hilfe der Dideoxy-Methode nach Sanger, F. und Coulson, A.R. "A rapid method for determining sequences in DNA by primed synthesis with DNA polymerase" J. Mol. Biol. 94, 441 (1975) ermittelt. Zu diesem Zweck wurden pBS-Subklone gemäß Beispiel 3 hergestellt und unter alkalischen Bedingungen in Einzelstrang-DNA's überführt.

Die Ergebnisse für die jeweiligen Klone sind im Sequenzprotokoll (Seq. ID-Nrn. 1 bis 5) dargestellt.

### Beispiel 8

### Herstellung von Antigenen über bakterielle Expressionsvektoren zur Induktion von Antikörpern

Die Protein-kodierenden Bereiche der in den gemäß Beispiel 3 hergestellten pBS-Subklonen insertierten cDNA-Klone HE 2 und HE 4 wurden mit Hilfe der Restriktionsendonukleasen BglI und PstI (HE 2) bzw. DdeI herausgeschnitten und in getrennten Ansätzen auf üblichem Wege (Sambrook et al., "Molecular Cloning - A Laboratory Manual", 2. Auflage, Cold Spring Harbor Laboratory Press (1989)) in den Expressions-Plasmidvektor pET3 (Rosenberg et al., "Vectors for selective expression of cloned DNAs by T7 RNA Polymerase", Gene, 56, 125-135 (1987)) unter Berücksichtigung der jeweiligen Leseraster umkloniert. Dieser Vektor ermöglicht die Insertion der verschiedenen cDNA-Inserts direkt hinter (stromabwärts) eine für das Enzym T7-Polymerase spezifischen Promotorsequenz.

Nach Transformation dieser Konstrukte in den E. coli Stamm BL21 (DE3), welcher über das Resistenzgen für Tetracyclin und über das Gen für die T7-Polymerase verfügt, erfolgte die Anzucht der Transformanten unter Tetracyclinselektion. Durch Zugabe von Rifampicin wurden alle E. coli eigenen Polymerasen inaktiviert. Da lediglich die T7-Polymerase gegen dieses Antibiotikum resistent ist und die erfindungsgemäßen cDNA-Inserts der Kontrolle des Promotors für die T7-Polymerase unterstanden, wurden primär die erfindungsgemäßen Polypeptide exprimiert.

Die auf diese Weise hergestellten bakteriogenen Polypeptide wurden anschließend aus den Bakterienextrakten gelelektrophoretisch aufgereinigt und direkt als Antigene für eine Immunisierung von Kaninchen und Hühnern verwendet.

### Beispiel 9

**Herstellung von Antigenen über chemische Synthese zur Induktion von Antikörpern**

Die Sequenz -Asp-Lys-Glu-Gly- (Seq. ID-Nr. 6) wurde per Computer als mögliches antigenes Epitop des HE 4-Polypeptids (Seq. ID-Nr. 4) identifiziert und zusammen mit den flankierenden Aminosäuren in der Gesamtsequenz-Asn-Asp-Lys-Glu-Gly-Ser-Ala-Pro-Gln-Val-Asn-Ile-Asn-Phe-(Seq. ID-Nr. 7) nach dem Verfahren von Merrifield (J.M. Stewart, "Synthesis and use of neuropeptides", Neuroendocrine Peptide Methodology, ed. P.M. Conn, Academic Press, New York, S. 815-844 (1989)) chemisch synthetisiert, wobei ein Cystein an Position 81 der Sequenz von NE 4 (Seq. ID-Nr. 4) durch ein Alanin ersetzt wurde.

In analoger Weise wurde ein potentiell immunogenes Epitop von HE 1 (Seq. ID-Nr. 1) bestimmt und einschließlich flankierender Aminosäuren in der Gesamtsequenz -Gln-Lys-Asp-Lys-Thr-Tyr-Ser-Tyr-Leu-Pro-Val-Lys-Ser-Glu-Tyr-Pro- (Seq. ID-Nr. 8) chemisch synthetisiert.

Die auf diese Weise hergestellten immunogenen Fragmente der Polypeptide HE 1 und HE 4 wurden anschließend jeweils mit m-Maleimidobenzoicsäure-N-hydroxysuccinamid-ester (M8759, Sigma) (Sambrook et al., "Molecular Cloning - A Laboratory Manual", 2. Auflage, Cold Spring Harbor Laboratory Press (1989)) an Trägersubstanzen (Keyhole-Limpet-Hämocyanin, KLH) gekoppelt und als Antigene für die Immunisierung von Kaninchen zur Erzeugung von Antikörpern eingesetzt.
SEQ ID NO: 1
   ART DER SEQUENZ: Nucleotid mit ensprechendem Protein
   SEQUENZLÄNGE: 816 Basenpaare
   STRANGFORM: Einzelstrang
   TOPOLOGIE: linear
   ART DES MOLEKÜLS: c-DNA zu m-RNA
   URSPRÜNGLICHE HERKUNFT ORGANISMUS: Mensch
   GEWEBE: Nebenhoden
   UNMITTELBARE EXPERIMENTELLE HERKUNFT:
   cDNA-Bibliothek aus humanem Nebenhoden
   - MERKMALE:: von 11 bis 87 BP Signalpeptid (S)
   von 88 bis 483 BP reifes Peptid (S)
   von 788 bis 797 BP Polyadenylierungssignal (S)
   EIGENSCHAFTEN: gewebespezifisches Sekretprotein des humanen Nebenhodens HE1 
ggattcccgg atg 13
Met
-19

cgt ttc ctg gca gct aca ttc ctg ctc ctg gcg 66
Arg Phe Leu Ala Ala Thr Phe Leu Leu Leu Ala
-8

ctc agc acc gct gcc cag gcc gaa ccg gtg cag 99
Leu Ser The Ala Ala Gln Ala Glu Pro Val Gln
-1 1 4

ttc aag gac tgc ggt tct gtg gat gga gtt ata 132
Phe Lys Asp Cys Gly Ser Val Asp Gly Val Ile
15

aag gaa gtg aat gtg agc cca tgc ccc acc caa 165
Lys Glu Val Gln Val Ser Pro Cys Pro Thr Gln
26

ccc tgc cag ctg agc aaa gga cag tct tac agc 198
Pro Cys Gln Leu Ser Lys Gly Gln Ser Tyr Ser
37

gtc aat gtc acc ttc acc agc aat att cag tct 231
Val Asn Val Thr Phe Thr Ser Asn Ile Gln Ser
48

aaa agc agc aag gcc gtg gtg cat ggc atc ctg 264
Lys Ser Ser Lys Ala Val Val His Gly Ile Leu
59
 
atg ggc gtc cca gtt ccc ttt ccc att cct gag 297
Met Gly Val Pro Val Pro Phe Pro Ile Pro Glu
70

cct gat ggt tgt aag agt gga att aac tgc cct 330
Pro Asp Gly Cys Lys Ser Gly Ile Asn Cys Pro
81

atc caa aaa gac aag acc tat agc tac ctg aat 363
Ile Gln Lys Asp Lys Thr Tyr Ser Tyr Leu Asn
92

aaa cta cca gtg aaa agc gaa tat ccc tct ata 396
Lys Leu Pro Val Lys Ser Glu Tyr Pro Ser Ile
103

aaa ctg gtg gtg gag tgg caa ctt cag gat gac 429
Lys Leu Val Val Glu Trp Gln Leu Gln Asp Asp
114

aaa aac caa agt ctc ttc tgc tgg gaa atc cca 462
Lys Asn Gln Ser Leu Phe Cys Trp Glu Ile Pro
125

gta cag atc gtt tct cat ctc taa gtgcctcatt 496
Val Gln Ile Val Ser His Leu ***
132

gagttcggtg catctggcca atgagtctgc tgagactctt 536

gacagcacct ccagctctgc tgcttcaaca acagtgactt 576

gctctccaat ggtatccagt gattcgttga agaggaggtg 616

ctctgtagca gaaactgagc tccgggtggc tggttctcag 656

tggttgtctc atgtctcttt ttctgtctta ggtggtttca 696

ttaaatgcag cacttggtta gcagatgttt aatttttttt 736

tttaacaaca ttaacttgtg gcctctttct acacctggaa 776

atttactctt gaataaataa aaactcgttt gtcttgtaaa 816
SEQ ID NO: 2
   ART DER SEQUENZ: Nucleotid
   SEQUENZLÄNGE: 736 Basenpaare
   STRANGFORM: Einzelstrang
   TOPOLOGIE: linear
   ART DES MOLEKÜLS: c-DNA zu m-RNA
   URSPRÜNGLICHE HERKUNFT ORGANISMUS: Mensch
   GEWEBE: Nebenhoden
   UNMITTELBARE EXPERIMENTELLE HERKUNFT:
   cDNA-Bibliothek aus humanem Nebenhoden
   - MERKMALE:: von 99 bis 164 BP Signalpeptid (S)
   165 bis 407 BP reifes Peptid (S)
   652 bis 657 BP Polyadenylierungssignal (S)
   EIGENSCHAFTEN: gewebespezifisches Sekretprotein des humanem Nebenhodens HE2 
GATGAC 6

AT GTATTTGCAC AAAAATTCTC AGGCACATTA AATTATT 45

GTA AACTGAAGTA AAACCTGGGT GCTTTCTGGC TTGCAG 84

TGCT CTTGGCAGAC ATG AGG CAA CGA TTG CTC CCG 119
Met Arg Gln Arg Leu Leu Pro
-22 -20

TCC GTC ACC AGC CTT CTC CTT GTG GCC CTG CTG 152
Ser Val Thr Ser Leu Leu Leu Val Ala Leu Leu
-10

TTT CCA GGA TCG TCT CAA GCC AGA CAT GTG AAC 185
Phe Pro Gly Ser Ser Gln Ala Arg His Val Asn
-1 1

CAC TCA GCC ACT GAG GCT CTC GGA GAA CTC AGG 218
His Ser Ala Thr Glu Ala Leu Gly Glu Leu Arg
10

GAA AGA GCC CCT GGG CAA GGC ACA AAC GGG TTT 251
Glu Arg Ala Pro Gly Gln Gly Thr Asn Gly Phe
20

CAG CTG CTA CGC CAC GCA GTG AAA CGG GAC CTC 284
Gln Leu Leu Arg His Ala Val Lys Arg Asp Leu
30 40

TTA CCA CCG CGC ACC CCA CCT TAC CAA GTG CAC 317
Leu Pro Pro Arg Thr Pro Pro Tyr Gln Val His
50
 
ATC TCT CAC CAG GAG GCT CGA GGA CCC TCA TTT   350
Ile Ser His Gln Glu Ala Arg Gly Pro Ser Phe
                                        60

AAG ATC TGC GTG GGC TTT TTA GGG CCT AGA TGG   383
Lys Ile Cys Val Gly Phe Leu Gly Pro Arg Trp
                                70

GCC AGG GGA TGT TCC ACT GGG AAT TAG ATACCAT   417
Ala Arg Gly Cys Ser Thr Gly Asn ***
                            80  81

CTG CCGTATGCAG CAAGGGATCT GCAGACTTTT TTTCTG   456

CCAT TCTGGTGAGA AAAAGCGTGA CATTTGCTCT GATCC   495

CTGGA ATAGGTGTTG CGTATCAAAT ACAGATGAAG AAGG   534

AAAAGA GAAACCAGAG ATGGATGGCA GATCTGGGAT CTA   573

AAATATA AGCTCCCGGA AGGCAGGGAT GTTGAAGTAT CC   612

CAAGGGCT TAAAGGAATG TGTGGCTTAT AGTAGGTGTT C   651

AATAAAATAT TTGTTGAATG AATTTAGMCC AAAGGTGAAG   700

AGCTGATAAA AGAGATTTTT TTAACTTCAA AAAAAA   736
SEQ ID NO: 3
   ART DER SEQUENZ: Nucleotid
   SEQUENZLÄNGE: 438 Basenpaare
   STRANGFORM: Einzelstrang
   TOPOLOGIE: linear
   ART DES MOLEKÜLS: c-DNA zu m-RNA
   URSPRÜNGLICHE HERKUNFT ORGANISMUS: Mensch
   GEWEBE: Nebenhoden
   UNMITTELBARE EXPERIMENTELLE HERKUNFT:
   cDNA-Bibliothek aus humanem Nebenhoden
   MERKMALE: von 411 bis 417 BP Polyadenylierungssignal (S)
   EIGENSCHAFTEN: gewebespezifische mRNA des humanen Nebenhodens HE3 
gaattccatt gtagcatgga cgggtatgtt gatagcatag 40
aagacctaaa gatggtagaa cctatcggca actagaaagt 80
ctatgcacat cctcaggtat tggtagagta ttcagtgctt 120
tctaagtagc annccctgcc tccatcaata gtcctaccac 160
tcccctcttg catttatttg tcaatgtttt ccaaatactt 200
agagttatga atagcataat ttcttgatac cataacnnna 240
gcctgtgttg tttcnntgcc tggaatacaa tttttgtctt 280
catttaccta atttactctt atacttttgt cgacattcag 320
ctcatatggc atctgttctt gattaaccca agactttcct 360
gatattgact ctcttgatac atacccaagc tgaacaatct 400
tcccttccct aaataaatta tatgactgca aaaaaaaa 438
SEQ ID NO: 4
   ART DER SEQUENZ: Nucleotid mit ensprechendem Protein
   SEQUENZLÄNGE: 590 Basenpaare
   STRANGFORM: Einzelstrang
   TOPOLOGIE: linear
   ART DES MOLEKÜLS: c-DNA zu m-RNA
   URSPRÜNGLICHE HERKUNFT ORGANISMUS: Mensch
   GEWEBE: Nebenhoden
   UNMITTELBARE EXPERIMENTELLE HERKUNFT:
   cDNA-Bibliothek aus humanem Nebenhoden
   - MERKMALE:: von 27 bis 106 BP Signalpeptid (S)
   von 107 bis 401 BP reifes Peptid (S)
   von 554 bis 557 BP Polyadenylierungssignal (S)
   EIGENSCHAFTEN: gewebespezifisches Sekretprotein des humanen Nebenhodens HE4 
cccctgc accccgcccg gcatagcacc 26

atg cct gct tgt cgc cta ggc ccg cta gcc gcc 59
Met Pro Ala Cys Arg Leu Gly Pro Leu Ala Ala
-30 -20

gcc ctc ctc ctc agc ctg ctg ctg ttc ggc ttc 92
Ala Leu Leu Leu Ser Leu Leu Leu Phe Gly Phe
-9

acc cta gtc tca ggc aca gga gca gag aag act 125
Thr Leu Val Ser Gly Thr Gly Ala Glu Lys Thr
-1 1 3

ggc gtg tgc ccc gag ctc cag gct gac cag aac 158
Gly Val Cys Pro Glu Leu Gln Ala Asp Gln Asn
14

tgc acg caa gag tgc gtc tcg gac agc gaa tgc 191
Cys Thr Gln Glu Cys Val Ser Asp Ser Glu Cys
25

gcc gac aac ctc aag tgc tgc agc gcg ggc tgt 224
Ala Asp Asn Leu Lys Cys Cys Ser Ala Gly Cys
36

gcc acc ttc tgc ctt ctc tgc ccc aat gat aag 257
Ala Thr Phe Cys Leu Leu Cys Pro Asn Asp Lys
47

gag ggt tcc tgc ccc cag gtg aac att aac ttt 290
Glu Gly Ser Cys Pro Gln Val Asn Ile Asn Phe
58
 
ccc cag ctc ggc ctc tgt cgg gac cag tgc cag     323
Pro Gln Leu Gly Leu Cys Arg Asp Gln Cys Gln
                                        69

gtg gac acg cag tgt cct ggc cag atg aaa tgc     356
Val Asp Thr Asn Cys Pro Gly Gln Met Lys Cys
                                        80

tgc cgc aat ggc tgt ggg aag gtg tcc tgt gtc     389
Cys Arg Asn Gly Cys Gly Lys Val Ser Cys Val
                                        91

act ccc aat ttc tga   ggtccagcca ccaccaggct     424
Thr Pro Asn Phe ***
            95

gagcagtgag gagagaaagt ttctgcctgg ccctgcatct     464

ggttccagcc cacctgccct cccctttttc gggactctgt     508

attccctctt ggggtgacca cagcttctcc ctttcccaac     552

caataaagta accactttca gcaaaaaaaa aaaaaaaa     590
SEQ ID NO: 5
   ART DER SEQUENZ: Nucleotid mit ensprechendem Protein
   SEQUENZLÄNGE: 544 Basenpaare
   STRANGFORM: Einzelstrang
   TOPOLOGIE: Linear
   ART DES MOLEKÜLS: c-DNA zu m-RNA
   URSPRÜNGLICHE HERKUNFT ORGANISMUS: Mensch
   GEWEBE: Nebenhoden
   UNMITTELBARE EXPERIMENTELLE HERKUNFT:
   cDNA-Bibliothek aus humanem Nebenhoden
   - MERKMALE:: von 45 bis 138 BP Signalpeptid (S)
   139 bis 282 BP reifes Peptid (S)
   504 bis 509 BP Polyadenylierungssignal (S)
   EIGENSCHAFTEN: gewebespezifisches Sekretprotein des humanem Nebenhodens HE5 
gaca gccacgaaga tcctaccaaa 44

atg aag cgc ttc ctc ttc ctc cta ctc acc atc 88
Met Lys Arg Phe Leu Phe Leu Leu Leu Thr Ile
-24 -20

acc ctc ctg gtt atg gta cag ata caa act gga 132
Thr Leu Leu Val Met Val Gln Ile Gln Thr Gly
-10

ctc tca gga caa aac gac tac cag cca aac cag 176
Leu Ser Gly Gln Asn Asp Tyr Gln Pro Asn Gln
-1 1

cag ccc ctc agc atc cag cag cat gag cga ggc 220
Gln Pro Leu Ser Ile Gln Gln His Glu Arg Gly
10 20

att ttc ctt ttc ttc gtg gcc aat gcc ata atc 264
Ile Phe Leu Phe Phe Val Ala Asn Ala Ile Ile
30

cac ctc ttc tgc ttc agt tga ggtgacacgt ctcag 308
His Leu Phe Cys Phe Ser ***
36

cctta gccctgtgcc ccctgaaaca gctgccacca tcact 352

cgcaa gagaatcccc ttcatctttg ggaggggttg atgcc 396

agaca tcaccaggtt gtagaagttt gacaggcagt gcatg 440

gggca acagcaaaat agggggtaa tgatgtaggg gccaa 484

gcagt gccxcagctg ggggaataaa gttacccttg tactg 528

caaaa aaaaaaaaaa 544
SEQ ID NO: 6
   ART DER SEQUENZ: AMINOSÄURESEQUENZ
   SEQUENZLÄNGE: 4 AMINOSÄUREN
   UNMITTELBARE EXPERIMENTELLE HERKUNFT: SEQ ID NO 4
   EIGENSCHAFTEN: ANTIGENES EPITOP DES HE 4-POLYPEPTIDS 
Asp Lys Glu Gly
1 2 3 4
SEQ ID NO: 7
   ART DER SEQUENZ: AMINOSÄURESEQUENZ
   SEQUENZLÄNGE: 14 AMINOSÄUREN
   UNMITTELBARE EXPERIMENTELLE HERKUNFT: SEQ ID NO 4
   EIGENSCHAFTEN: ANTIGENES EPITOP DES HE 4-POLYPEPTIDS MIT FLANKIERENDEN SEQUENZEN 
Asn Asp Lys Glu Gly Ser Ala Pro Gln Val
 1               5                   10

Asn Ile Asn Phe
            14
SEQ ID NO: 8
   ART DER SEQUENZ: AMINOSÄURESEQUENZ
   SEQUENZLÄNGE: 16 AMINOSÄUREN
   UNMITTELBARE EXPERIMENTELLE HERKUNFT
   EIGENSCHAFTEN: ANTIGENES EPITOP DES HE 1-POLYPEPTIDS

Gln Lys Asp Lys Thr Tyr Ser Tyr Leu Pro
1               5                   10

Val Lys Ser Glu Tyr Pro
                15

## Patentansprüche (Patentansprüche für folgende(n) Vertragsstaat(en): AT, BE, CH, LI, DE, DK, FR, GB, IT, LU, NL, SE)

1. Humanes, Epididymis-spezifisches Polypeptid, welches die in Seq. ID-Nr. 2 angegebene Aminosäuresequenz 1 bis 81 aufweist, sowie allelische Varianten oder Fragmente dieser Sequenz mit gleicher Gewebespezifität und Immunogenität.

2. DNA-Sequenzen, welche für die Polypeptide gemäß Anspruch 1 kodieren.

3. DNA-Sequenzen, ausgewählt aus
a) der in Seq. ID-Nr. 2 angegebenen Nukleotidsequenz,
b) Nukleotidsequenzen, welche die Basenpaare 99 bis 407 oder 165 bis 407 der Seq. ID-Nr. 2 einschließen, und
c) Nukleotidsequenzen, welche unter Berücksichtigung der Degeneration des genetischen Codes mit den Nukleotidsequenzen gemäß a) oder b) übereinstimmen.

4. Komplementärsequenzen zu den Nukleotidsequenzen nach den Ansprüchen 2 oder 3.

5. Verwendung von
a) Nukleotidsequenzen nach den Ansprüchen 2 bis 4 oder deren Komplementärsequenzen,
b) Fragmenten der Nukleotidsequenzen gemäß a), oder
c) Nukleotidsequenzen, die mit den Sequenzen gemäß a) oder b) hybridisieren,
als Sonden zum Auffinden der DNA-Sequenzen nach den Ansprüchen 2 bis 4 oder zur in situ-Hybridisierung bei der Gewebediagnostik von Biopsieproben oder Dünnschnitten.

6. Vektor-Moleküle, **dadurch gekennzeichnet,** daß sie eine der Sequenzen nach den Ansprüchen 2 bis 4 unter Aufrechterhaltung der Fähigkeit zur Replikation in geeigneten Wirtszellen insertiert enthalten.

7. Vektor-Moleküle nach Anspruch 6, **dadurch gekennzeichnet,** daß sie die Sequenzen in solcher Weise insertiert enthalten, daß deren Expression in geeigneten Wirtsorganismen erfolgen kann.

8. Prokaryontische oder eukaryontische Wirtszellen, **dadurch gekennzeichnet,** daß sie mit Vektor-Molekülen nach den Ansprüchen 6 oder 7 transformiert sind.

9. Wirtszellen nach Anspruch 8, **dadurch gekennzeichnet,** daß es sich um Säuger-Zellen handelt.

10. Verfahren zur Herstellung humaner, Epididymis-spezifischer Polypeptide, **dadurch gekennzeichnet,** daß man Wirtszellen gemäß Anspruch 8 oder 9 unter Bedingungen kultiviert, welche die Expression der Polypeptide erlauben, und man das Expressionsprodukt aus dem Kulturansatz gewinnt.

11. Antikörper, **dadurch gekennzeichnet,** daß sie in der Lage sind, eine Immunreaktion mit einem der Polypeptide nach Anspruch 1 einzugehen.

12. Antikörper nach Anspruch 11, **dadurch gekennzeichnet,** daß sie monoklonale Antikörper sind.

13. Antikörper nach Anspruch 11 oder 12, **dadurch gekennzeichnet,** daß sie mit einem Marker versehen sind.

14. Arzneimittel, **dadurch gekennzeichnet,** daß es ein Polypeptid gemäß Anspruch 1 oder Antikörper gemäß einem der Ansprüche 11 bis 13 als aktive Wirkstoffe enthält.

15. Mittel zur Diagnose von Störungen des Stoffwechsels der Epididymis-spezifischen Polypeptide gemäß Anspruch 1, **dadurch gekennzeichnet,** daß es Nukleotidsequenzen nach den Ansprüchen 2 bis 4 oder deren Komplementärsequenzen, oder Antikörper gemäß einem der Ansprüche 11 bis 13 enthält.

16. Verwendung von
a) Nukleotidsequenzen nach den Ansprüchen 2 bis 4 oder deren Komplementärsequenzen,
b) Fragmenten der Nukleotidsequenzen gemäß a), oder
c) Nukleotidsequenzen, die mit den Sequenzen gemäß a) oder b) hybridisieren
zur Herstellung vom Mitteln zur Diagnose von Störungen des Stoffwechsels der Epididymis-spezifischen Polypeptide gemäß Anspruch 1.

## Patentansprüche (Patentansprüche für folgende(n) Vertragsstaat(en): GR, ES)

1. Humanes, Epididymis-spezifisches Polypeptid, welches die in Seq. ID-Nr. 2 angegebene Aminosäuresequenz 1 bis 81 aufweist, sowie allelische Varianten oder Fragmente dieser Sequenz mit gleicher Gewebespezifität und Immunogenität.

2. DNA-Sequenzen, welche für die Polypeptide gemäß Anspruch 1 kodieren.

3. DNA-Sequenzen, ausgewählt aus
a) der in Seq. ID-Nr. 2 angegebenen Nukleotidsequenz,
b) Nukleotidsequenzen, welche die Basenpaare 99 bis 407 oder 165 bis 407 der Seq. ID-Nr. 2 einschließen, und
c) Nukleotidsequenzen, welche unter Berücksichtigung der Degeneration des genetischen Codes mit den Nukleotidsequenzen gemäß a) oder b) übereinstimmen.

4. Komplementärsequenzen zu den Nukleotidsequenzen nach den Ansprüchen 2 oder 3.

5. Verwendung von
a) Nukleotidsequenzen nach den Ansprüchen 2 bis 4 oder deren Komplementärsequenzen,
b) Fragmenten der Nukleotidsequenzen gemäß a), oder
c) Nukleotidsequenzen, die mit den Sequenzen gemäß a) oder b) hybridisieren,
als Sonden zum Auffinden der DNA-Sequenzen nach den Ansprüchen 2 bis 4 oder zur in situ-Hybridisierung bei der Gewebediagnostik von Biopsieproben oder Dünnschnitten.

6. Vektor-Moleküle, **dadurch gekennzeichnet,** daß sie eine der Sequenzen nach den Ansprüchen 2 bis 4 unter Aufrechterhaltung der Fähigkeit zur Replikation in geeigneten Wirtszellen insertiert enthalten.

7. Vektor-Moleküle nach Anspruch 6, **dadurch gekennzeichnet,** daß sie die Sequenzen in solcher Weise insertiert enthalten, daß deren Expression in geeigneten Wirtsorganismen erfolgen kann.

8. Prokaryontische oder eukaryontische Wirtszellen, **dadurch gekennzeichnet,** daß sie mit Vektor-Molekülen nach den Ansprüchen 6 oder 7 transformiert sind.

9. Wirtszellen nach Anspruch 8, **dadurch gekennzeichnet,** daß es sich um Säuger-Zellen handelt.

10. Verfahren zur Herstellung humaner, Epididymis-spezifischer Polypeptide, **dadurch gekennzeichnet,** daß man Wirtszellen gemäß Anspruch 8 oder 9 unter Bedingungen kultiviert, welche die Expression der Polypeptide erlauben, und man das Expressionsprodukt aus dem Kulturansatz gewinnt.

11. Antikörper, **dadurch gekennzeichnet,** daß sie in der Lage sind, eine Immunreaktion mit einem der Polypeptide nach Anspruch 1 einzugehen.

12. Antikörper nach Anspruch 11, **dadurch gekennzeichnet,** daß sie monoklonale Antikörper sind.

13. Antikörper nach Anspruch 11 oder 12, **dadurch gekennzeichnet,** daß sie mit einem Marker versehen sind.

14. Verfahren zur Herstellung eines Arzneimittels, **dadurch gekennzeichnet,** daß man ein Polypeptid gemäß Anspruch 1 oder Antikörper gemäß einem der Ansprüche 11 bis 13 als aktive Wirkstoffe zu einem Arzneimittel formuliert.

15. Mittel zur Diagnose von Störungen des Stoffwechsels der Epididymis-spezifischen Polypeptide gemäß Anspruch 1, **dadurch gekennzeichnet,** daß es Nukleotidsequenzen nach den Ansprüchen 2 bis 4 oder deren Komplementärsequenzen, oder Antikörper gemäß einem der Ansprüche 11 bis 13 enthält.

16. Verwendung von
a) Nukleotidsequenzen nach den Ansprüchen 2 bis 4 oder deren Komplementärsequenzen,
b) Fragmenten der Nukleotidsequenzen gemäß a), oder
c) Nukleotidsequenzen, die mit den Sequenzen gemäß a) oder b) hybridisieren
zur Herstellung vom Mitteln zur Diagnose von Störungen des Stoffwechsels der Epididymus-spezifischen Polypeptide gemäß Anspruch 1.

## Claims (Claims for the following Contracting State(s): AT, BE, CH, LI, DE, DK, FR, GB, IT, LU, NL, SE)

1. Human epididymis-specific polypeptide showing the amino acid sequence 1 to 81 given in Seq. ID. No. 2, and allelic variants or fragments of this sequence with an identical tissue specificity and immunogeneity.

2. DNA sequences encoding the polypeptides according to Claim 1.

3. DNA sequences selected from amongst
a) the nucleotide sequence given in Seq. ID. No. 2,
b) nucleotide sequences which include the base pairs 99 to 407 or 165 to 407 of Seq. ID. No. 2, and
c) nucleotide sequences which, taking into consideration the degeneration of the genetic code, agree with the nucleotide sequences in accordance with a) or b).

4. Complementary sequences to the nucleotide sequences according to Claims 2 or 3.

5. Use of
a) nucleotide sequences according to Claims 2 to 4 or their complementary sequences,
b) fragments of the nucleotide sequences in accordance with a), or
c) nucleotide sequences which hybridize with the sequences in accordance with a) or b)
as probes for locating the DNA sequences according to Claims 2 to 4 or for the in-situ hybridization in tissue diagnostics of biopsy specimens or thin sections.

6. Vector molecules, characterized in that they contain inserted one of the sequences according to Claims 2 to 4 while maintaining the capability of replicating in suitable host cells.

7. Vector molecules according to Claim 6, characterized in that they contain the sequences inserted in such a manner that they can be expressed in suitable host organisms.

8. Prokaryotic or eukaryotic host cells, characterized in that they are transformed with vector molecules according to Claims 6 or 7.

9. Host cells according to Claim 8, characterized in that they are mammalian cells.

10. Process for the preparation of human epididymis-specific polypeptides, characterized in that host cells according to Claim 8 or 9 are cultured under conditions which permit expression of the polypeptides, and obtaining the expression product from the culture batch.

11. Antibodies, characterized in that they are capable of undergoing an immune reaction with one of the polypeptides according to Claim 1.

12. Antibodies according to Claim 11, characterized in that they are monoclonal antibodies.

13. Antibodies according to Claim 11 or 12, characterized in that they are labelled.

14. Pharmaceutical, characterized in that it comprises a polypeptide according to Claim 1 or antibodies according to any of Claims 11 to 13 as active ingredients.

15. Composition for diagnosing metabolic disorders of the epididymis-specific polypeptides according to Claim 1, characterized in that it comprises nucleotide sequences according to Claims 2 to 4 or their complementary sequences or antibodies according to any of Claims 11 to 13.

16. Use of
a) nucleotide sequences according to Claims 2 to 4 or their complementary sequences,
b) fragments of the nucleotide sequences in accordance with a), or
c) nucleotide sequences which hybridize with the sequences in accordance with a) or b)
for the preparation of compositions for diagnosing metabolic disorders of the epididymis-specific polypeptides according to Claim 1.

## Claims (Claims for the following Contracting State(s): ES, GR)

1. Human epididymis-specific polypeptide showing the amimo acid sequence 1 to 81 given in Seq. ID. No. 2, and allelic variants or fragments of this sequence with an identical tissue specificity and immunogeneity.

2. DNA sequences encoding the polypeptides according to Claim 1.

3. DNA sequences selected from amongst
a) the nucleotide sequence given in Seq. ID. No. 2,
b) nucleotide sequences which include the base pairs 99 to 407 or 165 to 407 of Seq. ID. No. 2, and
c) nucleotide sequences which, taking into consideration the degeneration of the genetic code, agree with the nucleotide sequences in accordance with a) or b).

4. Complementary sequences to the nucleotide sequences according to Claims 2 or 3.

5. Use of
a) nucleotide sequences according to Claims 2 to 4 or their complementary sequences,
b) fragments of the nucleotide sequences in accordance with a), or
c) nucleotide sequences which hybridize with the sequences in accordance with a) or b)
as probes for locating the DNA sequences according to Claims 2 to 4 or for the in-situ hybridization in tissue diagnostics of biopsy specimens or thin sections.

6. Vector molecules, characterized in that they contain inserted one of the sequences according to Claims 2 to 4 while maintaining the capability of replicating in suitable host cells.

7. Vector molecules according to Claim 6, characterized in that they contain the sequences inserted in such a manner that they can be expressed in suitable host organisms.

8. Prokaryotic or eukaryotic host cells, characterized in that they are transformed with vector molecules according to Claims 6 or 7.

9. Host cells according to Claim 8, characterized in that they are mammalian cells.

10. Process for the preparation of human epididymis-specific polypeptides, characterized in that host cells according to Claim 8 or 9 are cultured under conditions which permit expression of the polypeptides, and obtaining the expression product from the culture batch.

11. Antibodies, characterized in that they are capable of undergoing an immune reaction with one of the polypeptides according to Claim 1.

12. Antibodies according to Claim 11, characterized in that they are monoclonal antibodies.

13. Antibodies according to Claim 11 or 12, characterized in that they are labelled.

14. Process for the preparation of a pharmaceutical, characterized in that a polypeptide according to Claim 1 or antibodies according to any of Claims 11 to 13 are formulated as active ingredients to a pharmaceutical.

15. Composition for diagnosing metabolic disorders of the epididymis-specific polypetides according to Claim 1, characterized in that it comprises nucleotide sequences according to Claims 2 to 4 or their complementary sequences or antibodies according to any of Claims 1 to 13.

16. Use of
a) nucleotide sequences according to Claims 2 to 4 or their complementary sequences,
b) fragments of the nucleotide sequences in accordance with a), or
c) nucleotide sequences which hybridize with the sequences in accordance with a) or b)
for the preparation of compositions for diagnosing metabolic disorders of the epididymis-specific polypeptides according to Claim 1.

## Revendications (Revendications pour l'(les) Etat(s) contractant(s) suivant(s): AT, BE, CH, LI, DE, DK, FR, GB, IT, LU, NL, SE)

1. Polypeptide humain, spécifique de l'épididyme, présentant la séquence d'aminoacides 1 à 81 indiquée dans SEQ ID n° 2, ainsi que des variants alléliques ou des fragments de cette séquence à même spécificité tissulaire et même immunogénicité.

2. Séquences d'ADN codant pour les polypeptides selon la revendication 1.

3. Séquence d'ADN choisie parmi
a) la séquence nucléotidique indiquée dans SEQ ID n° 2,
b) des séquences nucléotidiques qui comprennent les paires de bases 99 à 407 ou 165 à 407 de SEQ ID n° 2, et
c) des séquences nucléotidiques qui correspondent, d'après la dégénérescence du code génétique, à la séquence nucléotidique selon a) ou b).

4. Séquences complémentaires des séquences nucléotidiques selon la revendication 2 ou 3.

5. Utilisation
a) de séquences nucléotidiques selon les revendications 2 à 4 ou de leurs séquences complémentaires,
b) de fragments des séquences nucléotidiques selon a) ou
c) de séquences nucléotidiques qui s'hybrident avec la séquence selon a) ou b),
en tant que sondes pour la détection des séquences d'ADN selon les revendications 2 à 4 ou pour l'hybridation in situ dans le diagnostic tissulaire de coupes minces ou d'échantillons obtenus par biopsie.

6. Molécules de vecteur, caractérisées en ce qu'elles contiennent insérée l'une des séquences selon les revendications 2 à 4, les dites molécules vecteur étant capable de la réplication dans des cellules hôtes appropriées.

7. Molécules de vecteur selon la revendication 6, caractérisées en ce qu'elles contiennent les séquences insérées de telle façon que leur expression peut s'effectuer dans des organismes hôtes appropriés.

8. Cellules hôtes procaryotes ou eucaryotes, caractérisées en ce qu'elles sont transformées par des molécules de vecteur selon la revendication 6 ou 7.

9. Cellules hôtes selon la revendication 8, caractérisées en ce qu'il s'agit de cellules de mammifères.

10. Procédé pour la production de polypeptides humains, spécifiques de l'épididyme, caractérisé en ce que l'on cultive des cellules hôtes selon la revendication 8 ou 9, dans des conditions qui permettent l'expression des polypeptides, et on recueille le produit d'expression à partir du mélange de culture.

11. Anticorps, caractérisés en ce qu'ils peuvent intervenir dans une réaction immunitaire contre l'un des polypeptides selon la revendication 1.

12. Anticorps selon la revendication 11, caractérisés en ce qu'ils sont des anticorps monoclonaux.

13. Anticorps selon la revendication 11 ou 12, caractérisés en ce qu'ils sont munis d'un marqueur.

14. Médicament, caracterisé en ce qu'il contient comme substances actives un polypeptide selon la revendication ou des anticorps selon l'une des revendications 11 à 13.

15. Agent pour le diagnostic de troubles du métabolisme des polypeptides spécifiques d'épididyme selon la revendication 1, caractérisé en ce qu'il contient des séquences nucléotidiques selon les revendications 2 à 4 ou des séquences complémentaires de celles-ci, ou des anticorps selon l'une des revendications 11 à 13.

16. Utilisation
a) de séquences nucléotidiques selon les revendications 2 à 4 ou de leurs séquences complémentaires,
b) de fragments des séquences nucléotidiques selon a) ou
c) de séquences nucléotidiques qui s'hybrident avec la séquence selon a) ou b),
pour la préparation d'agents pour le diagnostic de troubles du métabolisme des polypeptides spécifiques de l'épididyme selon la revendication 1.

## Revendications (Revendications pour l'(les) Etat(s) contractant(s) suivant(s): ES, GR)

1. Polypeptide humain, spécifique de l'épididyme, présentant la séquence d'aminoacides 1 à 81 indiquée dans SEQ ID n° 2, ainsi que des variants alléliques ou des fragments de cette séquence à même spécificité tissulaire et même immunogénicité.

2. Séquences d'ADN codant pour les polypeptides selon la revendication 1.

3. Séquence d'ADN choisie parmi
a) la séquence nucléotidique indiquée dans SEQ ID n° 2,
b) des séquences nucléotidiques qui comprennent les paires de bases 99 à 407 ou 165 à 407 de SEQ ID n° 2, et
c) des séquences nucléotidiques qui correspondent, d'après la dégénérescence du code génétique, à la séquence nucléotidique selon a) ou b).

4. Séquences complémentaires des séquences nucléotidiques selon la revendication 2 ou 3.

5. Utilisation
a) de séquences nucléotidiques selon les revendications 2 à 4 ou de leurs séquences complémentaires,
b) de fragments des séquences nucléotidiques selon a) ou
c) de séquences nucléotidiques qui s'hybrident avec la séquence selon a) ou b),
en tant que sondes pour la détection des séquences d'ADN selon les revendications 2 à 4 ou pour l'hybridation in situ dans le diagnostic tissulaire de coupes minces ou d'échantillons obtenus par biopsie.

6. Molécules de vecteur, caractérisées en ce qu'elles contiennent insérée l'une des séquences selon les revendications 2 à 4, les dites molécules vecteur étant capable de la réplication dans des cellules hôtes appropriées.

7. Molécules de vecteur selon la revendication 6, caractérisées en ce qu'elles contiennent les séquences insérées de telle façon que leur expression peut s'effectuer dans des organismes hôtes appropriés.

8. Cellules hôtes procaryotes ou eucaryotes, caractérisées en ce qu'elles sont transformées par des molécules de vecteur selon la revendication 6 ou 7.

9. Cellules hôtes selon la revendication 8, caractérisées en ce qu'il s'agit de cellules de mammifères.

10. Procédé pour la production de polypeptides humains, spécifiques de l'épididyme, caractérisé en ce que l'on cultive des cellules hôtes selon la revendication 8 ou 9, dans des conditions qui permettent l'expression des polypeptides, et on recueille le produit d'expression à partir du mélange de culture.

11. Anticorps, caractérisés en ce qu'ils peuvent intervenir dans une réaction immunitaire contre l'un des polypeptides selon la revendication 1.

12. Anticorps selon la revendication 11, caractérisés en ce qu'ils sont des anticorps monoclonaux.

13. Anticorps selon la revendication 11 ou 12, caractérisés en ce qu'ils sont munis d'un marqueur.

14. Procédé pour la production d'un medicament, caractérisé en ce qu'un polypeptide selon la revendication 1 ou des anticorps selon l'une des revendication 11 à 13 sont formulés comme substances actives pour un medicament.

15. Agent pour le diagnostic de troubles du métabolisme des polypeptides spécifiques d'épididyme selon la revendication 1, caractérisé en ce qu'il contient des séquences nucléotidiques selon les revendications 2 à 4 ou des séquences complémentaires de celles-ci, ou des anticorps selon l'une des revendications 11 à 13.

16. Utilisation
a) de séquences nucléotidiques selon les revendications 2 à 4 ou de leurs séquences complémentaires,
b) de fragments des séquences nucléotidiques selon a) ou
c) de séquences nucléotidiques qui s'hybrident avec la séquence selon a) ou b),
pour la préparation d'agents pour le diagnostic de troubles du métabolisme des polypeptides spécifiques de l'épididyme selon la revendication 1.
